# EUROPEAN PATENT SPECIFICATION

(11) **EP 4 097 683 B1**
(45) Date of publication and mention of the grant of the patent: **30.04.2025**
(21) Application number: 21711762.1
(22) Date of filing: 01.03.2021
(51) Int. Cl.: G06T 11/00

(54) **METHODS AND APPARATUS FOR DEEP LEARNING BASED IMAGE ATTENUATION CORRECTION**
VERFAHREN UND VORRICHTUNG ZUR BILDDÄMPFUNGSKORREKTUR BASIEREND AUF TIEFENLERNEN
PROCÉDÉS ET APPAREIL DE CORRECTION D'ATTÉNUATION D'IMAGE BASÉE SUR UN APPRENTISSAGE PROFOND

(30) Priority: 04.03.2020 US 202062985120 P
(43) Date of publication of application: 07.12.2022
(73) Proprietor: Siemens Medical Solutions USA, Inc., Malvern, PA 19355 (US)
(72) Inventor: VAHLE, Thomas, 90409 Nürnberg (DE); FENCHEL, Matthias, 91054 Erlangen (DE)
(74) Representative: Horn Kleimann Waitzhofer Schmid-Dreyer Patent- und Rechtsanwälte PartG mbB
(86) International application number: PCT/EP2021/055044
(87) International publication number: WO 2021/175781

(56) References cited:
- CN-A- 110 809 782
- US-A1- 2020 126 231
- SPUHLER KARL D. ET AL: "Synthesis of Patient-Specific Transmission Data for PET Attenuation Correction for PET/MRI Neuroimaging Using a Convolutional Neural Network", THE JOURNAL OF NUCLEAR MEDICINE, vol. 60, no. 4, April 2019 (2019-04-01), US, pages 555 - 560, XP055813345, ISSN: 0161-5505, DOI: 10.2967/jnumed.118.214320
- DONGHWI HWANG ET AL: "Generation of PET Attenuation Map for Whole-Body Time-of-Flight 18 F-FDG PET/MRI Using a Deep Neural Network Trained with Simultaneously Reconstructed Activity and Attenuation Maps", THE JOURNAL OF NUCLEAR MEDICINE, vol. 60, no. 8, 25 January 2019 (2019-01-25), US, pages 1183 - 1189, XP055753286, ISSN: 0161-5505, DOI: 10.2967/jnumed.118.219493

## Description

### FIELD

Aspects of the present disclosure relate in general to medical diagnostic systems and, more particularly, to reconstructing images from nuclear imaging systems for diagnostic and reporting purposes.

### BACKGROUND

The article "Synthesis of Patient-Specific Transmission Data for PET Attenuation Correction for PET/MRI Neuroimaging Using a Convolutional Neural Network" (Spuhler Karl D. et al., The Journal of Nuclear Medicine, April 1, 2019) discloses a training of a convolutional neural network to generate patient-specific transmission data from T1 weighted MRI for attenuation correction in PET.
Nuclear imaging systems can employ various technologies to capture images. For example, some nuclear imaging systems employ positron emission tomography (PET) to capture images. PET is a nuclear medicine imaging technique that produces tomographic images representing the distribution of positron emitting isotopes within a body. Some nuclear imaging systems employ computed tomography (CT), for example, as a co-modality. CT is an imaging technique that uses x-rays to produce anatomical images. Magnetic Resonance Imaging (MRI) is an imaging technique that uses magnetic fields and radio waves to generate anatomical and functional images. Some nuclear imaging systems combine images from PET and CT scanners during an image fusion process to produce images that show information from both a PET scan and a CT scan (*e.g.,* PET/CT systems). Similarly, some nuclear imaging systems combine images from PET and MRI scanners to produce images that show information from both a PET scan and an MRI scan.

Typically, these nuclear imaging systems capture measurement data, and process the captured measurement data using mathematical algorithms to reconstruct medical images. For example, reconstruction can be based on the models that can include analytic or iterative algorithms or, more recently, deep learning algorithms. These conventional models, however, can have several drawbacks. Many of these nuclear imaging systems, for example, have high memory and computational requirements to reconstruct a medical image. Moreover, many image formation processes employed by at least some of these systems rely on approximations to compensate for detection loss. The approximations, however, can cause inaccurate and lower quality medical images. As such, there are opportunities to address deficiencies in nuclear imaging systems.

### SUMMARY

Systems and methods for generating attenuation maps based on background radiation to reconstruct medical images are disclosed.

The invention is set out in the appended set of claims.

### BRIEF DESCRIPTION OF THE DRAWINGS

The following will be apparent from elements of the figures, which are provided for illustrative purposes and are not necessarily drawn to scale.
FIG. 1 illustrates a nuclear image reconstruction system, in accordance with some embodiments.
FIG. 2 illustrates a block diagram of an example computing device that can perform one or more of the functions described herein, in accordance with some embodiments.
FIG. 3A illustrates a nuclear imaging system without a subject, in accordance with some embodiments.
FIG. 3B illustrates a nuclear imaging system with a subject, in accordance with some embodiments.
FIG. 4A illustrates exemplary portions of the nuclear image reconstruction system of FIG. 1, in accordance with some embodiments.
FIG. 4B illustrates exemplary portions of the nuclear image reconstruction system of FIG. 1, in accordance with some embodiments.
FIG. 5 is a flowchart of an example method to train a neural network, in accordance with some embodiments.
FIG. 6 is a flowchart of an example method to reconstruct an image, in accordance with some embodiments.

### DETAILED DESCRIPTION

This description of the exemplary embodiments is intended to be read in connection with the accompanying drawings, which are to be considered part of the entire written description.

The exemplary embodiments are described with respect to the claimed systems as well as with respect to the claimed methods. Furthermore, the exemplary embodiments are described with respect to methods and systems for image reconstruction, as well as with respect to methods and systems for training functions used for image reconstruction. Features, advantages, or alternative embodiments herein can be assigned to the other claimed objects and vice versa. For example, claims for the providing systems can be improved with features described or claimed in the context of the methods, and vice versa. In addition, the functional features of described or claimed methods are embodied by objective units of a providing system. Similarly, claims for methods and systems for training image reconstruction functions can be improved with features described or claimed in context of the methods and systems for image reconstruction, and vice versa.

Various embodiments of the present disclosure can employ machine learning methods or processes to provide clinical information from nuclear imaging systems. For example, the embodiments can employ machine learning methods or processes to reconstruct images based on captured measurement data, and provide the reconstructed images for clinical diagnosis. In some embodiments, machine learning methods or processes are trained, to improve the reconstruction of images.

Quantitative Positron Emission Tomography (PET) generally requires an attenuation map to calculate the number of photons that have either been lost for a sinogram bin (*i.e.*, attenuation correction) or wrongly assigned to another sinogram bin (*i.e.*, scatter correction). In systems that combine PET and computed tomography (CT), linear attenuation coefficients may be generated based on the CT images, and used to determine PET corrections. For a system which combines PET and magnetic resonance (MR), this is not possible and hence other methods need to be applied in order to correct the PET data for scatter and attenuation. Nonetheless, accurate attenuation/scatter correction is a fundamental requirement for state-of-the-art PET and PET/MR systems. These corrections allow for quantitative and artifact-free PET images that can be used for clinical diagnosis.

In some embodiments, background radiation generated by PET crystals of a PET/MR imaging system is detected. PET crystals can be located on a gantry of the PET/MR imaging system, and can include, for example, lutetium oxyorthosilicate scintillator (LSO) crystals or lutetium yttrium orthosilicate (LYSO) crystals. Further, a machine learning model, such as a neural network, can be trained to generate attenuation maps based on the detected background radiation and corresponding MR images. In some examples, during a PET/MR workflow, only a short MR sequence (*e.g*., a high-resolution Dixon VIBE protocol) is acquired and used as an input for the machine learning model to generate a transmission-based attenuation map. In some embodiments, the machine learning model can be trained based on radiation detected from PET measurement data and corresponding MR measurement data captured from a PET/MR system using volunteer subjects. In some embodiments, the machine learning model is trained and/or updated based on radiation detected from PET measurement data and corresponding MR measurement data captured for a patient. Once the machine learning model is trained, the PET/MR system can be employed for clinical imaging.

Among other advantages, the embodiments allow for the acquisition of ground-truth image data based on machine learning models trained on attenuation maps generated based on the detection of background radiation, and MR measurement data. For example, the embodiments may allow for crystal (*e.g*., LSO) background transmission scans and reconstruction using an MR prior image (*e.g*. Dixon scan) to improve low count rates and compute attenuation maps. As such, the machine learning model can be trained without providing a radiation dose to a subject. Moreover, in some examples, MR scan and deep learning neural network are employed to generate a transmission image from the MR as an attenuation map. In addition, in some examples, the embodiments allow a patient to be scanned with a PET/MR imaging system rather than a PET/CT imaging system. The patient may feel more comfortable with the PET/MR imaging system as whole-body MR scans can be performed with the patient's arms down, while CT scans may require the patient to hold their arms up. Moreover, although crystals in PET scanners are usually either made from LSO or LYSO, the embodiments can also be used any suitable PET crystals, independent of the crystal material, or with an independent source of radiation.

In some embodiments, a scanning device, such as a PET/MR scanner, provides PET measurement data, such as three-dimensional (3D) time-of-flight sinograms (*e.g*., measurement data). The PET/MR scanner can include crystal material, such as LSO or LYSO crystals, that, due to radioactive decay, emits gamma rays. For example, the PET/MR scanner can include crystal material along a gantry. The emitted gamma rays can be captured by other crystals, such as crystals along the gantry located across the emitting crystals, and detected by the PET/MR scanner. The PET/MR scanner can also detect gamma rays emitted from a patient being scanned. For example, the patient can be injected with radioactive material, where the radioactive material emits gamma rays that are captured by the crystals, and detected by the PET/MR scanner. The PET/MR scanner can provide PET measurement data to a computing device based on the detected gamma rays.

The PET/MR scanner can also capture MR images, and provide corresponding MR measurement data to the computing device. The computing device can reconstruct the MR images based on the MR measurement data, and provide the MR images to a trained neural network, such as a trained deep learning neural network. The trained neural network can generate an attenuation map (*e.g.,* a predicted attenuation map) based on the reconstructed MR image. Further, the computing device can generate an image volume (*e.g*., a 3 dimensional image) based on the generated attenuation map and the PET measurement data.

In some embodiments, the neural network is trained based on attenuation maps generated from PET measurement data, and reconstructed MR images generated from MR measurement data, where the PET measurement data and MR measurement data are received from the PET/MR scanner for one or more volunteers. In some examples, the volunteers are not injected with radioactive material. In some examples, the volunteers are injected with radioactive material.

As an example, the PET/MR scanner scans a volunteer who has not been injected with radioactive material. The PET/MR scanner generates PET measurement data based on PET scans of the volunteer (*e.g*., captured gamma rays as the PET/MR scanner scans the volunteer), and further generates MR measurement data (*e.g.,* an MRI sequence using high-resolution Dixon volume-interpolated breathhold examination (VIBE)) based on MR imaging scans of the volunteer. Because the volunteer was not injected with radioactive material, the PET images are generated based gamma rays captured from "background" radiation. The computing device receives the MR measurement data, and reconstructs an MR image based on the MR measurement data using any suitable method as known in the art.

Further, the computing device generates the attenuation maps based on the PET measurement data and a "background" radiation of the PET/MR scanner. To determine the "background" radiation, the PET/MR scanner is operated with no patient (*e.g.*, no patient on a patient table within the PET/MR scanner's field of view, blank scan), and the PET/MR scanner generates PET measurement data based on gamma rays generated by the crystal material of the PET/MR scanner itself. The computing device receives the PET measurement data identifying the captured "background" radiation, and stores the PET measurement data in memory. The PET measurement data can be captured for a period of time and aggregated in memory, and the computing device can determine a background level of radiation based on the aggregated PET measurement data. For example, the computing device can determine an average level of radiation as captured by various portions of crystal material along a gantry of the PET/MR scanner.

The computing device can then generate the attenuation maps based on the received PET measurement data and the determined background levels of radiation. The background level of radiation can be used as a "reference level" from which the attenuation correction as identified by the attenuation map is measured from. For example, the computing device can generate the attenuation maps based on a difference between the PET measurement data obtained for each of the volunteers and the PET measurement data identifying the background level of radiation. In some examples, the computing device generates the attenuation maps based on the PET measurement data obtained for each of the volunteers, the corresponding reconstructed MR images, and the PET measurement data identifying the background level of radiation. The reconstructed MR images can provide information about the shape of a person's body as well as tissue boundaries inside the patient, for example. As such, the embodiments may employ crystal background transmission scans and reconstruction using an MR prior image (*e.g*., Dixon scan) to improve low count rates and compute attenuation maps. In some examples, the embodiments employ a deep learning neural network to generate an attenuation map from an MR scan.

The attenuation correction for PET is not the only application for this approach, however. A similar problem can present itself during radiotherapy planning when using MR data. By adopting a final energy level, the described pipeline as well as the acquired data could be used for MR based radiotherapy planning as well.

In some examples, the computing device scales the generated attenuation maps to a corresponding energy window. For example the energy window may be defined by a lower energy value, and an upper energy value. The energy window is used to distinguish events from different processes (*e.g*., PET emission events from 375 to 650 keV) and transmission events (*e.g*., transmission events between a range of electronvolts, such as between 310 and 88 keV).

The computing device can then train the neural network based on the reconstructed MR images and corresponding attenuation maps. For example, the computing device may store a threshold amount of reconstructed MR images and corresponding attenuation maps generated for one or more volunteers within memory. Once the threshold amount of reconstructed MR images and corresponding attenuation maps is obtained, the computing device can retrieve the stored reconstructed MR images and corresponding attenuation maps from the memory, and train the neural network with the reconstructed MR images and corresponding attenuation maps. For training, the MR images can be labelled as input, and the corresponding attenuation maps can be labelled as output, for example. The neural network is trained to predict an attenuation map given a reconstructed MR image. For example, offline collection and training of the neural network may be based on pairs of MR and attenuation maps generated from background crystal transmissions (*e.g*., LSO or LYSO crystal transmissions). Once trained, online (*e.g*., with a real patient) prediction of attenuation maps from measured MR images can be based on the output from the trained neural network.

In some examples, multiple neural networks are trained based on one or more attributes of patients. For example, the reconstructed MR images and corresponding attenuation maps may be categorized according to one or more of a person's age, weight, height, and medical condition. As an example, a first neural network can be trained based on reconstructed MR images and corresponding attenuation maps generated for persons under the age of 16. In addition, a second neural network can be trained based on reconstructed MR images and corresponding attenuation maps generated for persons between the ages of 16 and 21, and a third neural network can be trained based on reconstructed MR images and corresponding attenuation maps generated for persons above the age of 21. During diagnosis of a patient, the appropriate neural network may be employed by the computing device to generate image volumes, as described herein. In addition, the additional parameters of age could be used as additional input parameters to one large network from a single combined training batch.

In some examples, the computing device validates the trained neural network during a validation period. For example, the computing device can apply the neural network to MR measurement data obtained from a validation test data set, generate a reconstructed MR image, and apply the trained neural network to the reconstructed MR image to generate a predicted attenuation map. The computing device can further determine a loss between the predicted attenuation map and an expected attenuation map (*e.g.*, the expected attenuation map could have been generated based on prior art processes). Training of the neural network can be complete with the loss has been minimized to at least a threshold.

Once trained, the computing device can apply the neural network to reconstructed MR images to generate attenuation maps (*e.g*., predicted attenuation maps). For example, the PET/MR scanner can capture MR scans and PET scans of a patient (*e.g.,* a patient injected with radioactive material), and can transmit corresponding MR measurement data and PET measurement data to the computing device. The computing device reconstructs an MR image based on the MR measurement data, and further applies the trained neural network to the reconstructed MR image to generate an attenuation map. The computing device the reconstructs an image volume based on the attenuation map and the reconstructed MR image. The computing device may display the image volume to a physician for evaluation and diagnosis, for example.

In some embodiments, a computing device generates an attenuation map for performing the attenuation correction of acquired PET measurement data. The computing device generates the attenuation map based on synthetic transmission images (*e.g*., synthetic 511 keV transmission images) captured from a PET system, such as a PET/MR system or PET/CT system, and background radiation determined based on blank scans.

In some examples, the computing device generates the synthetic transmission images using a trained neural network, such as a deep learning neural network. In some examples, the neural network is trained using co-registered, previously acquired MR and transmission images. In some examples, the synthetic transmission images are generated based on the background radiation generated by PET crystals of the PET system. In some examples, the PET crystals are LSO crystals or LYSO crystals. In some examples, the computing device reconstructs the background radiation based transmission images using corresponding MR images.

In some examples, the generated attenuation maps are applied to acquired PET measurement data (*e.g.,* PET emission data) to perform attenuation correction of the acquired PET measurement data, and to generate an attenuation corrected PET image. In some examples, the PET measurement data is acquired using the PET modality of a combined PET/MR system that allows acquisition of PET and MR measurement data. In some examples, the PET data is acquired using the PET modality of a combined PET/CT system that allows acquisition of PET and CT measurement data.

The attenuation correction for PET is not the only application for this approach, however. A similar problem can present itself during radiotherapy planning when using MR data. By adopting a final energy level, the described pipeline as well as the acquired data could be used for MR based radiotherapy planning as well.

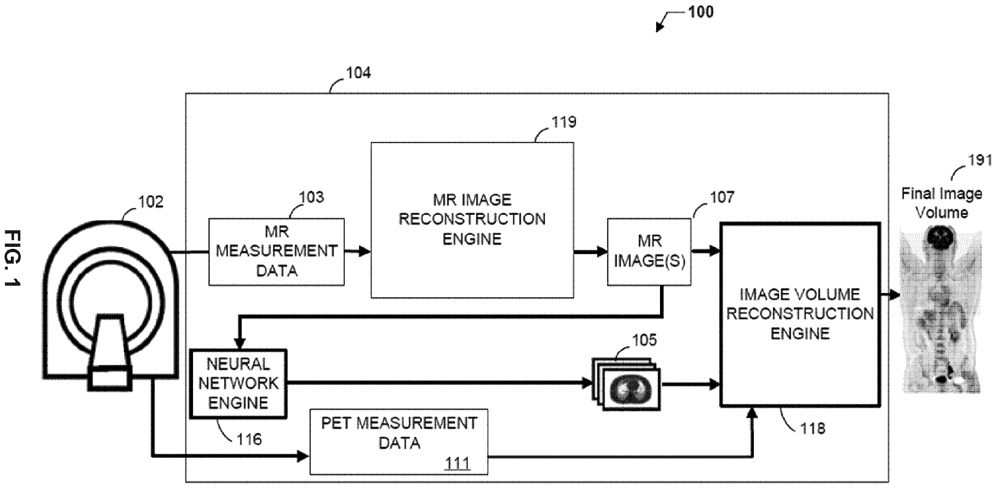

FIG. 1 illustrates one embodiment of a nuclear imaging system 100. As illustrated, nuclear imaging system 100 includes image scanning system 102 and image reconstruction system 104. Image scanning system 102 in this example is a PET/MR scanner, but in other examples, can be a PET/CT scanner (*e.g*., with CT as the corresponding co-modality instead of MR). Image scanning system 102 can capture MR images (*e.g.*, of a person), and generate MR measurement data 103 based on the MR scans. Image scanning system 102 can also capture PET images (*e.g.,* of the person), and generate PET measurement data 111 (*e.g.*, sinogram data) based on the captured PET images. The PET measurement data 111 can represent anything imaged in the scanner's field-of-view (FOV) containing positron emitting isotopes. For example, the PET measurement data 111 can represent whole-body image scans, such as image scans from a patient's head to thigh. Image scanning system 102 can transmit the MR measurement data 103 and the PET measurement data 111 to image reconstruction system 104.

In some examples, all or parts of image reconstruction system 104 are implemented in hardware, such as in one or more field-programmable gate arrays (FPGAs), one or more application-specific integrated circuits (ASICs), one or more state machines, one or more computing devices, digital circuitry, or any other suitable circuitry. In some examples, parts or all of image reconstruction system 104 can be implemented in software as executable instructions such that, when executed by one or more processors, cause the one or more processors to perform respective functions as described herein. The instructions can be stored in a non-transitory, computer-readable storage medium, for example.

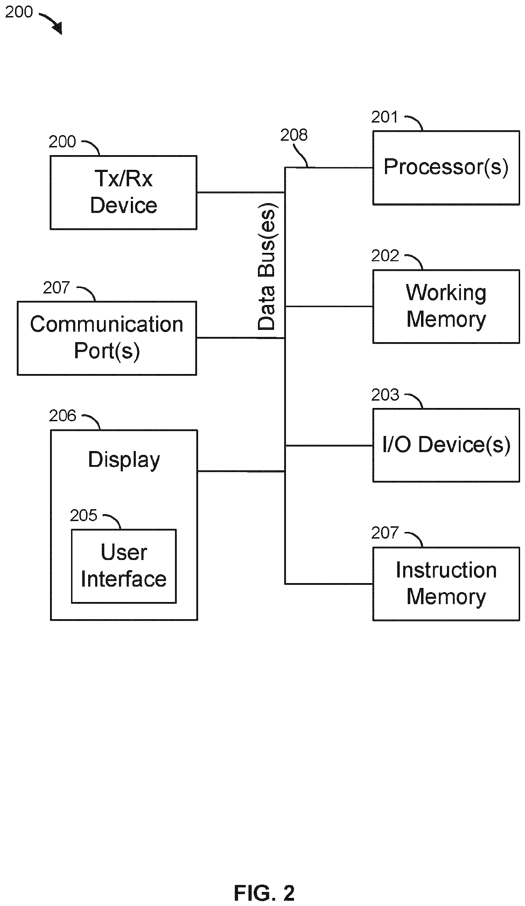

For example, FIG. 2 illustrates a computing device 200 that can be employed by the image reconstruction system 104. Computing device 200 can implement, for example, one or more of the functions of image reconstruction system 104 described herein.

Computing device 200 can include one or more processors 201, working memory 202, one or more input/output devices 203, instruction memory 207, a transceiver 204, one or more communication ports 207, and a display 206, all operatively coupled to one or more data buses 208. Data buses 208 allow for communication among the various devices. Data buses 208 can include wired, or wireless, communication channels.

Processors 201 can include one or more distinct processors, each having one or more cores. Each of the distinct processors can have the same or different structure. Processors 201 can include one or more central processing units (CPUs), one or more graphics processing units (GPUs), application specific integrated circuits (ASICs), digital signal processors (DSPs), and the like.

Processors 201 can be configured to perform a certain function or operation by executing code, stored on instruction memory 207, embodying the function or operation. For example, processors 201 can be configured to perform one or more of any function, method, or operation disclosed herein.

Instruction memory 207 can store instructions that can be accessed (*e.g*., read) and executed by processors 201. For example, instruction memory 207 can be a non-transitory, computer-readable storage medium such as a read-only memory (ROM), an electrically erasable programmable read-only memory (EEPROM), flash memory, a removable disk, CD-ROM, any non-volatile memory, or any other suitable memory. For example, instruction memory 207 can store instructions that, when executed by one or more processors 201, cause one or more processors 201 to perform one or more of the functions of image reconstruction system 104, such as one or more of the encoding segment 120 functions, one or more of the Radon inversion layer 140 functions, or one or more of the refinement and scaling segment 160 functions.

Processors 201 can store data to, and read data from, working memory 202. For example, processors 201 can store a working set of instructions to working memory 202, such as instructions loaded from instruction memory 207. Processors 201 can also use working memory 202 to store dynamic data created during the operation of computing device 200. Working memory 202 can be a random access memory (RAM) such as a static random access memory (SRAM) or dynamic random access memory (DRAM), or any other suitable memory.

Input-output devices 203 can include any suitable device that allows for data input or output. For example, input-output devices 203 can include one or more of a keyboard, a touchpad, a mouse, a stylus, a touchscreen, a physical button, a speaker, a microphone, or any other suitable input or output device.

Communication port(s) 207 can include, for example, a serial port such as a universal asynchronous receiver/transmitter (UART) connection, a Universal Serial Bus (USB) connection, or any other suitable communication port or connection. In some examples, communication port(s) 207 allows for the programming of executable instructions in instruction memory 207. In some examples, communication port(s) 207 allow for the transfer (e.g., uploading or downloading) of data, such as MRI measurement data 103 and attenuation maps 105.

Display 206 can display user interface 205. User interfaces 205 can enable user interaction with computing device 200. For example, user interface 205 can be a user interface for an application that allows for the viewing of final image volumes 191. In some examples, a user can interact with user interface 205 by engaging input-output devices 203. In some examples, display 206 can be a touchscreen, where user interface 205 is displayed on the touchscreen.

Transceiver 204 allows for communication with a network, such as a Wi-Fi network, an Ethernet network, a cellular network, or any other suitable communication network. For example, if operating in a cellular network, transceiver 404 is configured to allow communications with the cellular network. Processor(s) 401 is operable to receive data from, or send data to, a network via transceiver 204.

Referring back to FIG. 1, image reconstruction system 104 includes neural network engine 116, MR image reconstruction engine 119, and image volume reconstruction engine 118. MR image reconstruction engine 119 operates on MR measurement data 103 (*e.g.*, MR raw data) to generate reconstructed MR image 107. MR image reconstruction engine 119 can generate reconstructed MR images 107 based on corresponding MR measurement data 103 using any suitable method known in the art. Further, neural network engine 116 receives reconstructed MR images 107, and applies a trained neural network, such as a trained deep learning neural network as described herein, to the reconstructed MR images 107 to generate attenuation maps 105. For example, the neural network could have been trained based on reconstructed MR images and measured attenuation maps (*e.g*., ground truth data) during a training period, and further validated during a validation period (*e.g*., based on test data comprising MR images). The generated attenuation map 105 can identify density differences of a patient's body that can be used to correct for the absorption of photons emitted from radioactive decay (*e.g*., radioactive decay of crystal material of image scanning system 102).

Image volume reconstruction engine 118 obtains PET measurement data 111 (*e.g.,* PET raw data) and the generated attenuation map 105, and reconstructs a final image volume 191. For example, image volume reconstruction engine 118 applies the attenuation map 105 to PET measurement data 111 to generate the final image volume 191. Final image volume 191 can include image data that can be provided for display and analysis, for example.

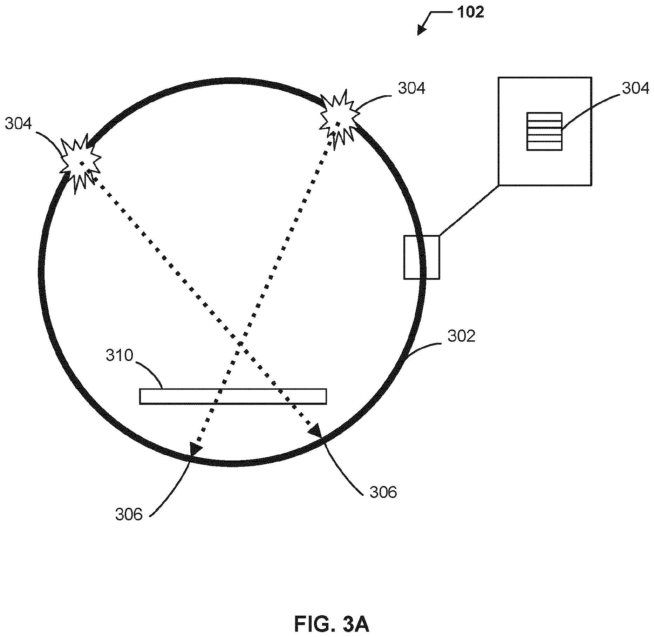

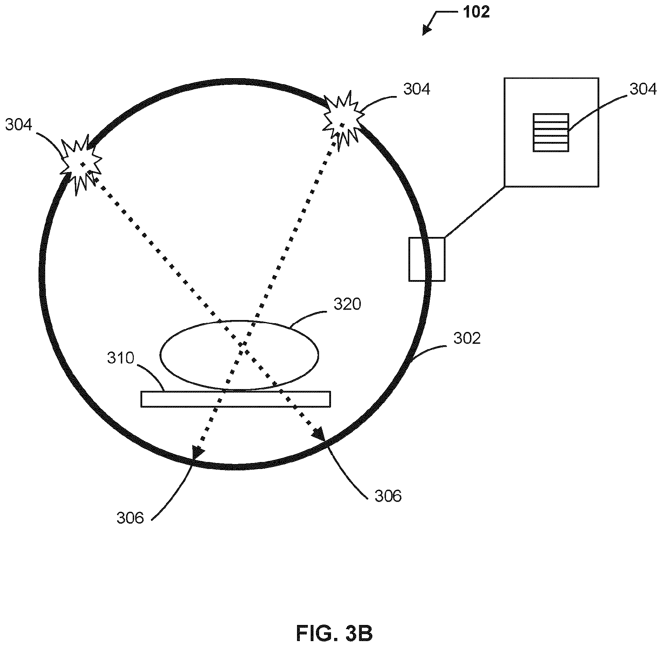

FIGS. 3A and 3B illustrate exemplary portions of image scanning system 102 including a gantry 302 and a patient table 310 located within the gantry 302. Gantry 302 may include crystal material 304, 306, such as LSO or LYSO crystals. Radioactive decay of crystal material 304 can cause gamma ray emissions, which can be detected by other crystal material 306. While FIG. 3B illustrates a patient 320 located on patient table 310, FIG. 3A includes no patient.

As described herein, image reconstruction system 104 can determine background levels of radiation generated by crystals 304 when no patient is located on patient table 310, as illustrated in FIG. 3A, based on gamma emissions captured by crystals 306. Further, to train a neural network, such as the neural network of neural network engine 116, image scanning system 102 captures MR scans and corresponding PET scans with patient 320 located on patient table 310, as illustrated in FIG. 3B. The patient 320 has no injected radioactivity, and thus detected activity (*e.g*., detected counts) is based on radioactive decay of crystals 304, 306. Image scanning system 102 can provide MR measurement data 103 and PET measurement data 111 to image reconstruction system 104 based on the MR scans and PET scans, respectively.

Image reconstruction system 104 can reconstruct MR images based on the MR measurement data 103, and generate attenuation maps, such as attenuation maps 105, based on the reconstructed MR images and the detected background levels of radiation. Image reconstruction system 104 can train a neural network, such as the neural network of neural network engine 116, based on matching pairs of the attenuation maps and reconstructed MR images.

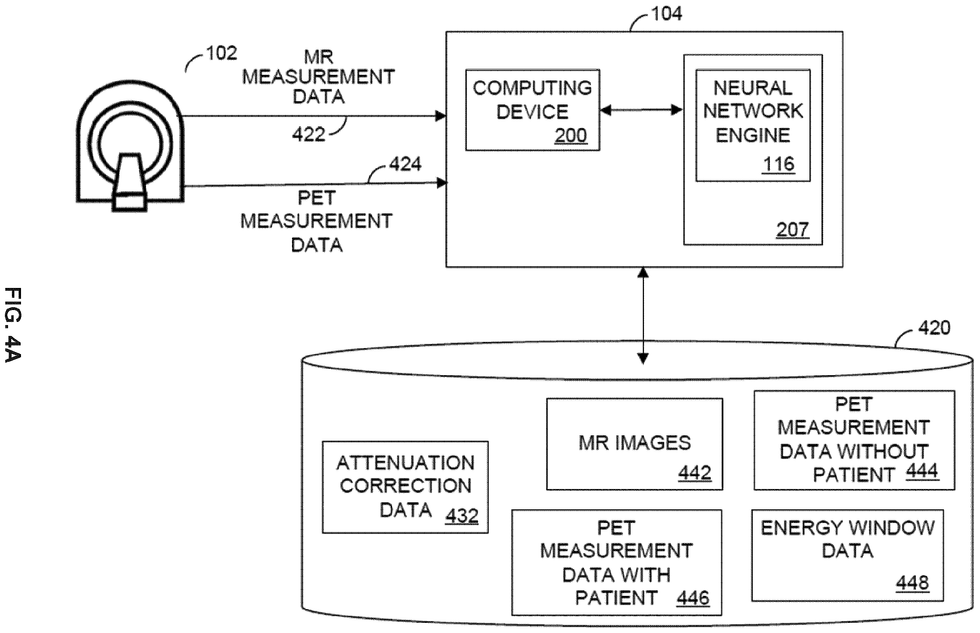

For example, FIG. 4A illustrates image reconstruction system 104 receiving MR measurement data 422 and PET measurement data 424 from image scanning system 102. Computing device 200 can reconstruct MR images 442 based on the received MR measurement data 422 according to any suitable method, and can store reconstructed MR images 442 in database 420. Database 420 can be a local or remote storage device, such as a cloud-based server, a disk (*e.g.*, a hard disk), a memory device on another application server, a networked computer, or any other suitable data storage device.

Further, image reconstruction system 104 can receive PET measurement data 424 when no patient is within image scanning system 102 (*e.g.*, blank scan as illustrated in FIG. 3A), and can store PET measurement data without patient 444 in database 420. Computing device 220 can determine a background level of radiation based on PET measurement data without patient 444. Further, image reconstruction system 104 can also receive PET measurement data 424 when a patient is within image scanning system 102 (*e.g.*, as illustrated in FIG. 3B), and store PET measurement data with patient 446 in database 420.

Computing device 200 can generate attenuation maps, such as attenuation maps 105, based on PER measurement data with patient 446 and a background level of radiation as identified by PET measurement data without patient 444. For example, computing device 200 can generate attenuation correction data 432 that identifies and characterizes the attenuation maps, and can store the attenuation correction data 432 within database 420. In some examples, computing device 200 generates the attenuation maps based on PER measurement data with patient 446, the background level of radiation as identified by PET measurement data without patient 444, and reconstructed MR images 422. The MR images 422 can provide information about a patient's body as well as tissue boundaries within the patient, for example. In some examples, computing device 200 scales the attenuation maps to a corresponding energy window identified by energy window data 448. The energy window may identify a range of electronvolts, such as 380 - 650 kev. For example, and based on energy window data 448, attenuation maps may be scaled to an energy level, such as 511 kev. Computing device 200 can train the neural network based on the generated attenuation maps and corresponding MR images 422.

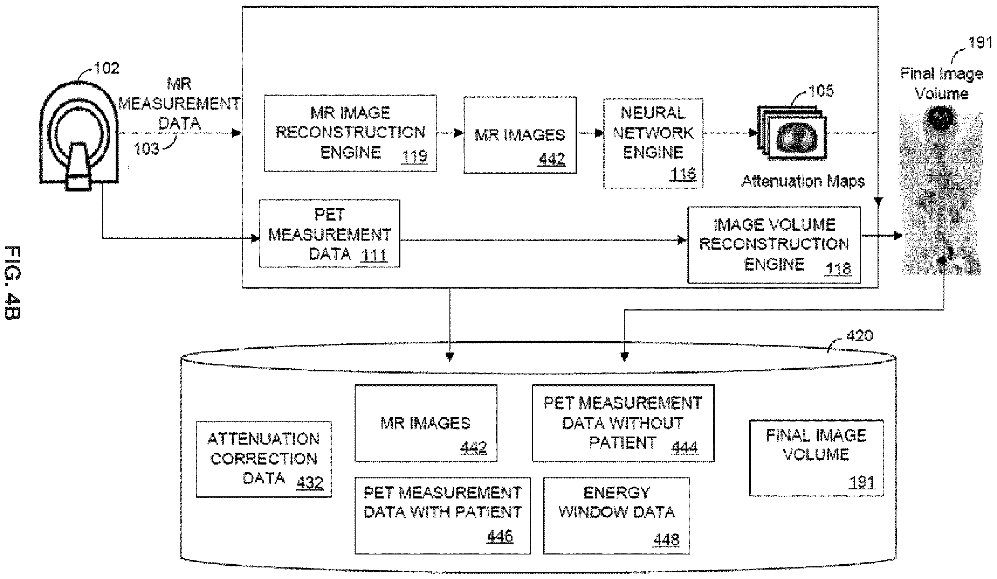

FIG. 4B illustrates the generation of a final image volume 191 based on a trained neural network. The trained neural network can generate a predicted attenuation map based on an MR image. As illustrated, MR image reconstruction engine 119 receives MR measurement data 103, and generates an MR image 422 according to any suitable method. Neural network engine 116 receives the MR image 442 from MR image reconstruction engine 119, and applies a trained neural network to MR image 422 to generate an attenuation map 105. Image volume reconstruction engine 118 receives PET measurement data 111 from 102, where the PET measurement data 111 corresponds to the received MR measurement data 103 (*e.g.*, PET measurement data 111 and MR measurement data 103 are based on simultaneous PET and MR scans, respectively, of a same person). Image volume reconstruction engine 118 further receives the generated attenuation map 105, and adjusts (*e.g*., corrects) PET measurement data 111 based on attenuation map 105 to generate the final image volume 191.

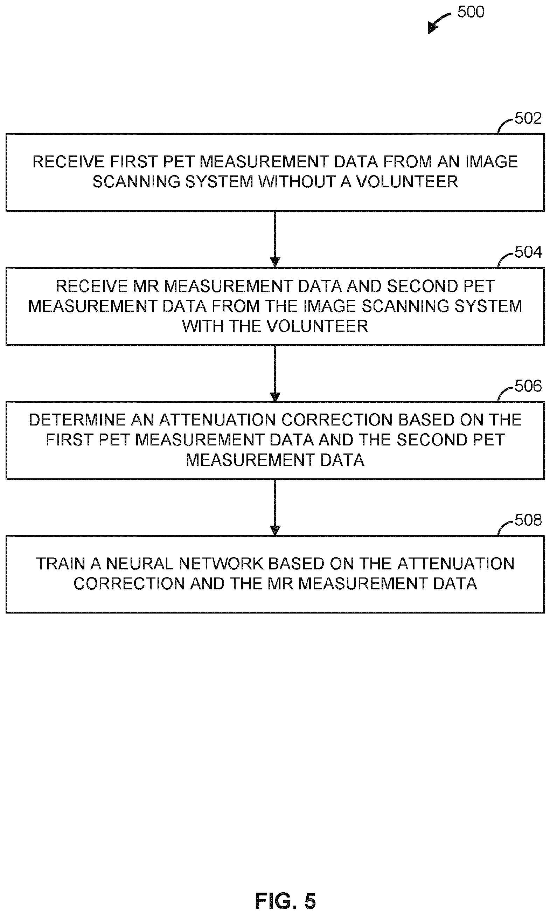

FIG. 5 is a flowchart of an example method 500 to train a neural network. The method can be performed by one or more computing devices, such as computing device 200. Beginning at step 502, first PET measurement data is received from an image scanning system. No volunteer (*e.g*., patient) is located within the image scanning system. For example, image reconstruction system 104 can receive the first PET measurement data, such as PET measurement data 111, from image scanning system 102. Image reconstruction system 104 can determine a background radiation level of the image scanning system based on the first PET measurement data. At step 504, MR measurement data and corresponding second PET measurement data is received from the image scanning system. The MR measurement data and corresponding second PET measurement data are captured with a volunteer located within the image scanning system. For example, image reconstruction system 104 can receive MR measurement data 103 and corresponding PET measurement data 111 from image scanning system 102 based on MR scans and PET scans performed for the volunteer.

Further, at step 506, an attenuation correction is determined based on the first PET measurement data (*e.g.*, the background radiation level) and the second PET measurement data. An attenuation map can identify the attenuation correction. For example, image reconstruction system 104 can generate an attenuation map 105 based on PET measurement data 111 and a previously determined background level of radiation of image scanning system 102, such as a background level identified by PET measurement data without patient 444 stored in database 420. At step 508, a neural network is trained based on the attenuation correction and the received MR measurement data. For example, image reconstruction system 104 can train a neural network of neural network engine 116 based on generated attenuation maps 105 and corresponding reconstructed MR images 107. In some examples, the trained neural network is stored in a database, such as database 420.

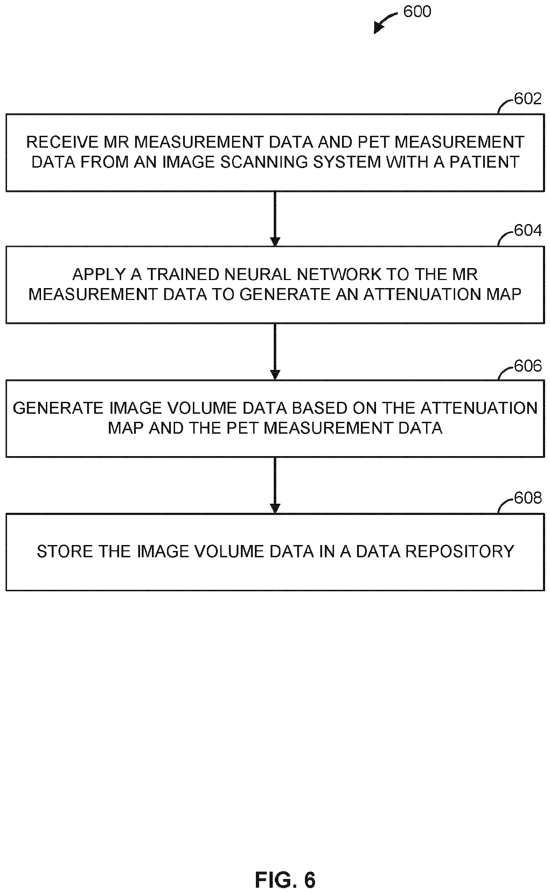

FIG. 6 is a flowchart of an example method 600 to generate an image volume, and can be carried out by one or more computing device such as, for example, computing device 200. Beginning at step 602, MR measurement data and PET measurement data (*e.g*., sinogram data) is received from an image scanning system. The MR measurement data and PET measurement data correspond to MR and PET scans of a patient. For example, image reconstruction system 104 can receive MR measurement data 103 and PET measurement data 111 from image scanning system 102 for a patient. At step 604, a trained neural network is applied to the MR measurement data to generate an attenuation map. The neural network could have been trained in accordance with method 500. As an example, neural network engine 116 can apply a trained neural network to reconstructed MR images 442 to generate attenuation map 105.

Proceeding to step 606, image volume data is generated based on the attenuation map and the received PET measurement data. The image volume data can identify and characterize an image volume (*e.g*., a 3D image volume). For example, image reconstruction system 104 can generate final image volume 191 based on attenuation maps 105 and corresponding PET measurement data 111. At step 608, the final image volume is stored in a database. For example, image reconstruction system 104 can store the generated final image volume 191 in database 420.

The apparatuses and processes are not limited to the specific embodiments described herein. In addition, components of each apparatus and each process can be practiced independent and separate from other components and processes described herein.

The previous description of embodiments is provided to enable any person skilled in the art to practice the disclosure. The various modifications to these embodiments will be readily apparent to those skilled in the art, and the generic principles defined herein can be applied to other embodiments without the use of inventive faculty. The present disclosure is not intended to be limited to the embodiments shown herein, but only by the scope of the appended set of claims.

## Claims

1. A computer-implemented method (500) comprising:
receiving (502) first positron emission tomography (PET) measurement data from an image scanning system (102), the first PET measurement data being obtained with no patient (320) within the image scanning system (102);
determining a reference level of radiation of the image scanning system (102) based on the first PET measurement data;
receiving (504) magnetic resonance (MR) measurement data and second PET measurement data from the image scanning system (102), the second PET measurement data being obtained with a patient (320) within the image scanning system (102);
generating a first attenuation map based on the first PET measurement data and the second PET measurement data;
training a neural network with the first attenuation map (105) and the MR measurement data; and
storing the trained neural network in a database (420).

2. The computer-implemented method of claim 1 further comprising:
receiving second MR measurement data from the image scanning system (102); and
applying the trained neural network to the second MR measurement data to determine a second attenuation map.

3. The computer-implemented method of claim 2 further comprising generating an image based on the second attenuation map.

4. The computer-implemented method of claim 1, wherein the second attenuation map is generated based on prior images computed using MR measurement data.

5. The computer-implemented method of claim 1 wherein the first attenuation map is generated based on synthetic transmission images.

6. The computer-implemented method of claim 1 further comprising generating the synthetic transmission images based on a detected background radiation generated by the image scanning system (102).

7. The computer-implemented method of claim 1 comprising scaling the first attenuation map based on a corresponding energy window.

8. The computer-implemented method of claim 1 wherein the neural network is a deep learning neural network.

9. A non-transitory computer readable medium storing instructions that, when executed by at least one processor (201), cause the at least one processor (201) to perform operations comprising:
receiving first positron emission tomography (PET) measurement data from an image scanning system (102), the first PET measurement data being obtained with no patient (320) within the image scanning system (102);
determining a reference level of radiation of the image scanning system (102) based on the first PET measurement data;
receiving magnetic resonance (MR) measurement data and second PET measurement data from the image scanning system (102), the second PET measurement data being obtained with a patient (320) within the image scanning system (102);
generating a first attenuation map based on the first PET measurement data and the second PET measurement data;
training a neural network with the first attenuation map and the MR measurement data; and
storing the trained neural network in a database (420).

10. The non-transitory computer readable medium of claim 9 storing instructions that, when executed by at least one processor (201), further cause the at least one processor (201) to perform operations comprising the method steps of claim 2, claims 2 and 3, claims 5 and claim 6, or claim 7.

11. The non-transitory computer readable medium of claim 9 wherein the second attenuation map is generated based on prior images computed using MR measurement data.

12. A system comprising:
a database (420); and
at least one processor (201) communicatively coupled to the database (420) and configured to:
receive first positron emission tomography (PET) measurement data from an image scanning system (102), the first PET measurement data being obtained with no patient (320) within the image scanning system (102);
determine a reference level of radiation of the image scanning system (102) based on the first PET measurement data;
receive magnetic resonance (MR) measurement data and second PET measurement data from the image scanning system (102), the second PET measurement data being obtained with a patient (320) within the image scanning system (102);
generate a first attenuation map based on the first PET measurement data and the second PET measurement data;
train a neural network with the first attenuation map and the MR measurement data; and
store the trained neural network **the** database (420).

13. The system of claim 12, wherein the at least one processor (201) is configured to perform the method steps of claim 2, claims 2 and 3, claims 5 and 6 or claim 7.

14. The system of claim 12, wherein the second attenuation map is generated based on prior images computed using MR measurement data.

## Patentansprüche

1. Computerimplementiertes Verfahren (500), das Folgendes umfasst:
Empfangen (502) erster Positronenemissionstomographie-Messdaten (PET-Messdaten) von einem Bildabtastsystem (102), wobei die ersten PET-Messdaten ohne Patienten (320) im Bildabtastsystem (102) erhalten wurden;
Bestimmen eines Bezugsstrahlungspegels des Bildabtastsystems (102) auf der Grundlage der ersten PET-Messdaten;
Empfangen (504) von Magnetresonanz-Messdaten (MR-Messdaten) und zweiten PET-Messdaten vom Bildabtastsystem (102), wobei die zweiten PET-Messdaten mit einem Patienten (320) im Bildabtastsystem (102) erhalten wurden;
Erzeugen eines ersten Dämpfungskennfelds auf der Grundlage der ersten PET-Messdaten und der zweiten PET-Messdaten;
Trainieren eines neuronalen Netzes mit dem ersten Dämpfungskennfeld (105) und den MR-Messdaten und
Speichern des trainierten neuronalen Netzes in einer Datenbank (420).

2. Computerimplementiertes Verfahren nach Anspruch 1, das ferner Folgendes umfasst:
Empfangen zweiter MR-Messdaten vom Bildabtastsystem (102) und
Anwenden des trainierten neuronalen Netzes auf die zweiten MR-Messdaten, um ein zweites Dämpfungskennfeld zu bestimmen.

3. Computerimplementiertes Verfahren nach Anspruch 2, das ferner ein Erzeugen eines Bilds auf der Grundlage des zweiten Dämpfungskennfelds umfasst.

4. Computerimplementiertes Verfahren nach Anspruch 1, wobei das zweite Dämpfungskennfeld auf der Grundlage vorhergehender Bilder erzeugt wird, die unter Verwendung von MR-Messdaten berechnet wurden.

5. Computerimplementiertes Verfahren nach Anspruch 1, wobei das erste Dämpfungskennfeld auf der Grundlage synthetischer Transmissionsbilder erzeugt wird.

6. Computerimplementiertes Verfahren nach Anspruch 1, das ferner ein Erzeugen der synthetischen Transmissionsbilder auf der Grundlage einer detektierten Hintergrundstrahlung umfasst, die durch das Bildabtastsystem (102) erzeugt wurde.

7. Computerimplementiertes Verfahren nach Anspruch 1, das ein Skalieren des ersten Dämpfungskennfelds auf der Grundlage eines entsprechenden Energiefensters umfasst.

8. Computerimplementiertes Verfahren nach Anspruch 1, wobei das neuronale Netz ein neuronales Netz mit tiefgehendem Lernen ist.

9. Nicht transitorisches computerlesbares Medium, das Befehle speichert, die dann, wenn sie durch mindestens einen Prozessor (201) ausgeführt werden, bewirken, dass der mindestens eine Prozessor (201) Operationen durchführt, die Folgendes umfassen:
Empfangen erster Positronenemissionstomographie-Messdaten (PET-Messdaten) von einem Bildabtastsystem (102), wobei die ersten PET-Messdaten ohne Patienten (320) im Bildabtastsystem (102) erhalten wurden;
Bestimmen eines Bezugsstrahlungspegels des Bildabtastsystems (102) auf der Grundlage der ersten PET-Messdaten;
Empfangen von Magnetresonanz-Messdaten (MR-Messdaten) und zweiten PET-Messdaten vom Bildabtastsystem (102), wobei die zweiten PET-Messdaten mit einem Patienten (320) im Bildabtastsystem (102) erhalten wurden;
Erzeugen eines ersten Dämpfungskennfelds auf der Grundlage der ersten PET-Messdaten und der zweiten PET-Messdaten;
Trainieren eines neuronalen Netzes mit dem ersten Dämpfungskennfeld und den MR-Messdaten und
Speichern des trainierten neuronalen Netzes in einer Datenbank (420).

10. Nicht transitorisches computerlesbares Medium nach Anspruch 9, das Befehle speichert, die dann, wenn sie durch mindestens einen Prozessor (201) ausgeführt werden, ferner bewirken, dass der mindestens eine Prozessor (201) Operationen ausführt, die die Verfahrensschritte nach Anspruch 2, den Ansprüchen 2 und 3, Anspruch 5 und Anspruch 6 oder Anspruch 7 umfassen.

11. Nicht transitorisches computerlesbares Medium nach Anspruch 9, wobei das zweite Dämpfungskennfeld auf der Grundlage vorhergehender Bilder erzeugt wird, die unter Verwendung von MR-Messdaten berechnet wurden.

12. System, das Folgendes umfasst:
eine Datenbank (420); und
mindestens einen Prozessor (201), der an die Datenbank (420) kommunikationstechnisch gekoppelt ist und konfiguriert ist zum:
Empfangen erster Positronenemissionstomographie-Messdaten (PET-Messdaten) von einem Bildabtastsystem (102), wobei die ersten PET-Messdaten ohne Patienten (320) im Bildabtastsystem (102) erhalten wurden;
Bestimmen eines Bezugsstrahlungspegels des Bildabtastsystems (102) auf der Grundlage der ersten PET-Messdaten;
Empfangen von Magnetresonanz-Messdaten (MR-Messdaten) und zweiten PET-Messdaten vom Bildabtastsystem (102), wobei die zweiten PET-Messdaten mit einem Patienten (320) im Bildabtastsystem (102) erhalten wurden;
Erzeugen eines ersten Dämpfungskennfelds auf der Grundlage der ersten PET-Messdaten und der zweiten PET-Messdaten;
Trainieren eines neuronalen Netzes mit dem ersten Dämpfungskennfeld und den MR-Messdaten und
Speichern des trainierten neuronalen Netzes in der Datenbank (420).

13. System nach Anspruch 12, wobei der mindestens eine Prozessor (201) konfiguriert ist, die Verfahrensschritte nach Anspruch 2, den Ansprüchen 2 und 3, den Ansprüchen 5 und 6 oder Anspruch 7 durchzuführen.

14. System nach Anspruch 12, wobei das zweite Dämpfungskennfeld auf der Grundlage vorhergehender Bilder erzeugt wird, die unter Verwendung von MR-Messdaten berechnet wurden.

## Revendications

1. Procédé (500) mis en œuvre par ordinateur, comprenant :
la réception (502) de premières données de mesure par tomographie par émission de positons (PET) en provenance d'un système d'acquisition d'images par balayage (102), les premières données de mesure PET étant obtenues en l'absence d'un patient (320) dans le système d'acquisition d'images par balayage (102) ;
la détermination d'un niveau de référence de rayonnement du système d'acquisition d'images par balayage (102) sur la base des premières données de mesure PET ;
la réception (504) de données de mesure par résonance magnétique (MR) et de deuxièmes données de mesure PET en provenance du système d'acquisition d'images par balayage (102), les deuxièmes données de mesure PET étant obtenues en présence d'un patient (320) dans le système d'acquisition d'images par balayage (102) ;
la génération d'une première carte d'atténuation sur la base des premières données de mesure PET et des deuxièmes données de mesure PET ;
l'entraînement d'un réseau neuronal à l'aide de la première carte d'atténuation (105) et des données de mesure MR ; et
le stockage du réseau neuronal entraîné dans une base de données (420).

2. Procédé mis en œuvre par ordinateur selon la revendication 1, comprenant en outre :
la réception de deuxièmes données de mesure MR en provenance du système d'acquisition d'images par balayage (102) ; et
l'application du réseau neuronal entraîné aux deuxièmes données de mesure MR afin de déterminer une deuxième carte d'atténuation.

3. Procédé mis en œuvre par ordinateur selon la revendication 2, comprenant en outre la génération d'une image sur la base de la deuxième carte d'atténuation.

4. Procédé mis en œuvre par ordinateur selon la revendication 1, dans lequel la deuxième carte d'atténuation est générée sur la base d'images précédentes calculées au moyen de données de mesure MR.

5. Procédé mis en œuvre par ordinateur selon la revendication 1, dans lequel la première carte d'atténuation est générée sur la base d'images de transmission synthétiques.

6. Procédé mis en œuvre par ordinateur selon la revendication 1, comprenant en outre la génération des images de transmission synthétiques sur la base d'un rayonnement de fond détecté généré par le système d'acquisition d'images par balayage (102).

7. Procédé mis en œuvre par ordinateur selon la revendication 1, comprenant la mise à l'échelle de la première carte d'atténuation sur la base d'une fenêtre d'énergie correspondante.

8. Procédé mis en œuvre par ordinateur selon la revendication 1, dans lequel le réseau neuronal est un réseau neuronal d'apprentissage profond.

9. Support non transitoire lisible par ordinateur stockant des instructions qui, lorsqu'elles sont exécutées par au moins un processeur (201), amènent l'au moins un processeur (201) à réaliser des opérations comprenant :
la réception de premières données de mesure par tomographie par émission de positons (PET) en provenance d'un système d'acquisition d'images par balayage (102), les premières données de mesure PET étant obtenues en l'absence d'un patient (320) dans le système d'acquisition d'images par balayage (102) ;
la détermination d'un niveau de référence de rayonnement du système d'acquisition d'images par balayage (102) sur la base des premières données de mesure PET ;
la réception de données de mesure par résonance magnétique (MR) et de deuxièmes données de mesure PET en provenance du système d'acquisition d'images par balayage (102), les deuxièmes données de mesure PET étant obtenues en présence d'un patient (320) dans le système d'acquisition d'images par balayage (102) ;
la génération d'une première carte d'atténuation sur la base des premières données de mesure PET et des deuxièmes données de mesure PET ;
l'entraînement d'un réseau neuronal à l'aide de la première carte d'atténuation et des données de mesure MR ; et
le stockage du réseau neuronal entraîné dans une base de données (420).

10. Support non transitoire lisible par ordinateur selon la revendication 9 stockant des instructions qui, lorsqu'elles sont exécutées par au moins un processeur (201), amènent en outre l'au moins un processeur (201) à réaliser des opérations comprenant les étapes de procédé selon la revendication 2, les revendications 2 et 3, la revendication 5 et la revendication 6, ou la revendication 7.

11. Support non transitoire lisible par ordinateur selon la revendication 9, dans lequel la deuxième carte d'atténuation est générée sur la base d'images précédentes calculées au moyen de données de mesure MR.

12. Système, comprenant :
une base de données (420) ; et
au moins un processeur (201) couplé en communication à la base de données (420) et configuré pour :
recevoir des premières données de mesure par tomographie par émission de positons (PET) en provenance d'un système d'acquisition d'images par balayage (102), les premières données de mesure PET étant obtenues en l'absence d'un patient (320) dans le système d'acquisition d'images par balayage (102) ;
déterminer un niveau de référence de rayonnement du système d'acquisition d'images par balayage (102) sur la base des premières données de mesure PET ;
recevoir des données de mesure par résonance magnétique (MR) et des deuxièmes données de mesure PET en provenance du système d'acquisition d'images par balayage (102), les deuxièmes données de mesure PET étant obtenues en présence d'un patient (320) dans le système d'acquisition d'images par balayage (102) ;
générer une première carte d'atténuation sur la base des premières données de mesure PET et des deuxièmes données de mesure PET ;
entraîner un réseau neuronal à l'aide de la première carte d'atténuation et des données de mesure MR ; et
stocker le réseau neuronal entraîné dans une base de données (420).

13. Système selon la revendication 12, dans lequel l'au moins un processeur (201) est configuré pour réaliser les étapes de procédé selon la revendication 2, les revendications 2 et 3, les revendications 5 et 6, ou la revendication 7.

14. Système selon la revendication 12, dans lequel la deuxième carte d'atténuation est générée sur la base d'images précédentes calculées au moyen de données de mesure MR.
